# EUROPÄISCHE PATENTSCHRIFT

(11) **EP 3 023 431 B1**
(45) Veröffentlichungstag und Bekanntmachung des Hinweises auf die Patenterteilung: **04.01.2017**
(21) Anmeldenummer: 14193779.7
(22) Anmeldetag: 19.11.2014
(51) Int. Cl.: C07H 13/06, C11D 1/83

(54) **Konzentrierte, niedrigviskose Rhamnolipid-Zusammensetzungen**
Concentrated, low viscosity rhamnolipid compounds
Compositions de rhamnolipides concentrées et peu visqueuses

(43) Veröffentlichungstag der Anmeldung: 25.05.2016
(73) Patentinhaber: Evonik Degussa GmbH, 45128 Essen (DE)
(72) Erfinder: Schilling, Martin, 53121 Bonn (DE); Kleinen, Jochen, 52525 Heinsberg (DE); Lorenz, Josef, 47809 Krefeld (DE); Wenk, Hans Henning, 45470 Mülheim an der Ruhr (DE)

(56) Entgegenhaltungen:
- DE-A1-102012 221 519
- YUTAKA ISHIGAMI ET AL: "Colloid chemical effect of polar head moieties of a rhamnolipid-type biosurfactant", LANGMUIR, Bd. 9, Nr. 7, 1. Juli 1993 (1993-07-01), Seiten 1634-1636, XP055190601, ISSN: 0743-7463, DOI: 10.1021/la00031a006

## Beschreibung

### Gebiet der Erfindung

Gegenstand der Erfindung sind konzentrierte Rhamnolipid-Zusammensetzungen sowie Verfahren zu ihrer Herstellung.

### Stand der Technik

Rhamnolipide sind grenzflächenaktive Glykolipide und Stoffwechselprodukte bestimmter Mikroorganismen. Sie verfügen über besondere tensidische Eigenschaften, wie z.B. starke Schaumbildung und sind für verschiedenste, technische Anwendungen interessant. Rhamnolipide können sowohl mit Wildstämmen als auch durch gentechnisch veränderte Mikroorganismen hergestellt werden. Verfahren zur fermentativen Herstellung und Aufarbeitung von Rhamnolipiden sind im Stand der Technik ausführlich beschrieben, so z.B. in der US20130130319.

Tenside, die in kosmetische Formulierungen (z.B. Shampoos, Handwaschmittel), Haushaltsreiniger sowie Geschirrspülmittel eingearbeitet werden, müssen bei gängigen Verarbeitungstemperaturen in flüssiger Form vorliegen, um die Verarbeitung über Rohrleitungssysteme und Pumpen beim Hersteller solcher Produkte zu gewährleisten. Die Viskosität sollte hierbei gering sein, um eine einfache und zuverlässige Förderung sicherzustellen. Gleichzeitig ist hierbei trotzdem eine möglichst hohe Tensidkonzentration erwünscht, um umweltfreundliche Formulierungen mit einem geringen Wassergehalt herstellen zu können. Gleichzeitig soll der Viskositätsabfall bei Verdünnung mit Wasser so gering wie möglich gehalten werden, weil die formulierten Endprodukte wie z.B. Shampoos, Handwaschmittel und Waschmittel eine ausreichende Viskosität aufweisen sollen, um für den Anwender besser handhabbar zu sein.
Aus der Literatur sind keine Rhamnolipid enthaltenden Zusammensetzungen bekannt, die diesen Anforderungen entsprechen.
Bei den aus der Literatur bekannten und den auf dem Markt verfügbaren Produktformen, handelt es sich entweder um Feststoffe die über verschiedene Verfahren in mehr oder weniger reiner Form isoliert wurden (z.B. Entfernung eines Lösungsmittels durch Trocknung), um reine Produkte, die in geringer und mittlerer wässriger Konzentration vermarktet werden (z.B. bis 25 Gew. %), oder um flüssige Produkte bei denen die Hauptkomponente(n) aus der Fermentation stammendes Pflanzenöl bzw. Abbauprodukte des Pflanzenöls ist/sind. Bei diesen handelt es sich somit um Rhamnolipid Lösungen in einer Ölphase, und die entsprechenden Produkte haben den entscheidenden Nachteil, dass sie nur noch sehr schlecht schäumen und somit für eigentliche tensidische Anwendungen uninteressant sind.
Aus der DE4237334A1 ist weiterhin eine hochkonzentrierte Produktform bekannt, die durch ein besonders einfaches Verfahren zur Aufreinigung und Aufkonzentrierung von Rhamnolipiden erhalten werden kann. Hierbei wird das RL durch Ansäuern auf pH =3 aus einer Fermentationsbrühe ausgefällt und der entsprechende RL Feststoff kann durch Zentrifugation aufkonzentriert werden. Hierbei werden Feststoffsuspensionen bzw. Pasten mit einem hohen Rhamnolipid-Feststoffgehalt von 30 bis 40 Gew.-% und sehr hoher Viskosität erhalten.
Abdel-Mawgoud et al. in Appl Biochem Biotechnol. 2009 May;157(2):329-45 beschreiben ein ähnliches Verfahren.
Die derart erhaltenen Feststoffdispersionen sind für eine industrielle, großtechnische Weiterverarbeitung, wie z.B. Herstellung von Formulierungen für Shampoos und Haushaltsreiniger ungeeignet, da sie sich für eine Förderung mit üblicherweise eingesetzten Pumpen aufgrund ihrer hohen Viskosität und ihrer Inhomogenität nicht eignen.
DE102012221519A1 beschreibt ein Verfahren zur Gewinnung von Rhamnolipiden mit hervorragenden Schaumeigenschaften, bei dem wässrige Rhamnolipidlösungen mit einem pH-Wert von 7 und einer Konzentration von ca. 50 Gew.-% erhalten werden. Ein Nachteil diese Produktform ist der unerwünschte, sehr starke Viskositätsabfall bei Verdünnung. Ein weiterer Nachteil ist die im Neutralen verringerte mikrobiologische Stabilität

Aufgabe der Erfindung war es, hochkonzentrierte und niedrigviskose Rhamnolipid enthaltende Zusammensetzungen bereitzustellen. Diese sollen weiterhin eine möglichst niedrige lonenstärke aufweisen und eine Verdünnung mit Wasser soll zu möglichst geringen Viskositätsänderungen führen.

### Beschreibung der Erfindung

Überraschenderweise wurde gefunden, dass besonders hochkonzentrierte und gleichzeitig niedrigviskose Rhamnolipid enthaltende Zusammensetzungen durch eine teilweise Neutralisation einer hochkonzentrierten und hochviskosen, sauren Rhamnolipid Feststoffsuspension/paste zu erhalten sind. Diese Zusammensetzungen zeigen überraschenderweise bei Verdünnung mit Wasser einen geringeren Viskositätsabfall als vollständig neutralisierte Zusammensetzungen. Für die Neutralisation können anorganische und organische Basen eingesetzt werden. Gegenstand der vorliegenden Erfindung sind daher wässrige Zusammensetzungen enthaltend hohe Konzentrationen von Rhamnolipiden. Ein weiterer Gegenstand der Erfindung ist ein Verfahren zur Herstellung von wässrigen Zusammensetzungen enthaltend hohe Konzentrationen von Rhamnolipiden.

Ein Vorteil der vorliegenden Erfindung ist, dass die lonenstärke der Rhamnolipid enthaltenden Zusammensetzungen klein gehalten werden kann. Hierdurch wird dem Formulierer entsprechende Flexibilität bei der nachträglichen Anpassung des pH-Wertes und des Salzgehaltes der finalen Endformulierung gelassen.
Ein weiterer Vorteil der vorliegenden Erfindung ist, dass die Zusammensetzungen eine erhöhte mikrobiologische Stabilität aufweisen.
Noch ein Vorteil der vorliegenden Erfindung ist, dass die Zusammensetzungen einfacher verdünnbar sind.
Ein weiterer Vorteil der vorliegenden Erfindung ist, dass die Zusammensetzungen mit anderen Tensiden gut mischbar sind.
Noch ein Vorteil der vorliegenden Erfindung ist, dass die Zusammensetzungen die Formulierung konzentrierter Tensidendformulierungen ("*concentrates*") ermöglichen. Ein weiterer Vorteil der vorliegenden Erfindung ist, dass die Zusammensetzungen aufgrund ihrer hohen Konzentration eine verringerte Schaumtendenz aufweisen, und somit Transport und Förderung vereinfacht werden.
Noch ein Vorteil der vorliegenden Erfindung ist, dass die Zusammensetzungen eine einfache Einarbeitung hydrophober Komponenten wie z. B. Öle erlauben.
Ein weiterer Vorteil der vorliegenden Erfindung ist, dass die Zusammensetzungen eine hohe Lagerstabiliät aufweisen.
Noch ein Vorteil der vorliegenden Erfindung ist, dass die Zusammensetzungen bei Verdünnung mit Wasser keine extremen Viskositätssprünge durchlaufen.

Ein weiterer Vorteil der vorliegenden Erfindung ist, dass die Zusammensetzungen bei ihrer Herstellung und ihrem Transport in Rohrleitung geringere Verschmutzungen verursachen und überdies hinaus eine einfachere Reinigung ermöglichen.
Noch ein Vorteil der vorliegenden Erfindung ist, dass die Zusammensetzungen zu ihrem Transport einen geringeren Energiebedarf zum Transport erfordern.

Beansprucht wird eine Zusammensetzung enthaltend
30 Gew.-% bis 70 Gew.-%, bevorzugt 35 Gew.-% bis 60 Gew.-%, besonders bevorzugt
40 Gew.-% bis 50 Gew.-%, mindestens eines Rhamnolipides und
30 Gew.-% bis 70 Gew.-%, bevorzugt 40 Gew.-% bis 65 Gew.-%, besonders bevorzugt
50 Gew.-% bis 60 Gew.-%, Wasser
wobei sich die Gewichtsprozente auf die Gesamtzusammensetzung beziehen, dadurch gekennzeichnet, dass der pH-Wert der Zusammensetzung bei 25 °C von 5,5 bis < 7,0, bevorzugt von 5,6 bis 6,2, besonders bevorzugt von 5,6 bis 6,0, beträgt.

Unter dem Begriff "Rhamnolipid" im Zusammenhang mit der vorliegenden Erfindung werden Rhamnolipide, deren protonierten Formen sowie insbesondere deren Salze mitumfasst.
Unter dem Begriff "Rhamnolipid" im Zusammenhang mit der vorliegenden Erfindung werden insbesondere Mischungen aus Verbindungen der allgemeinen Formel (I) und deren Salze verstanden, 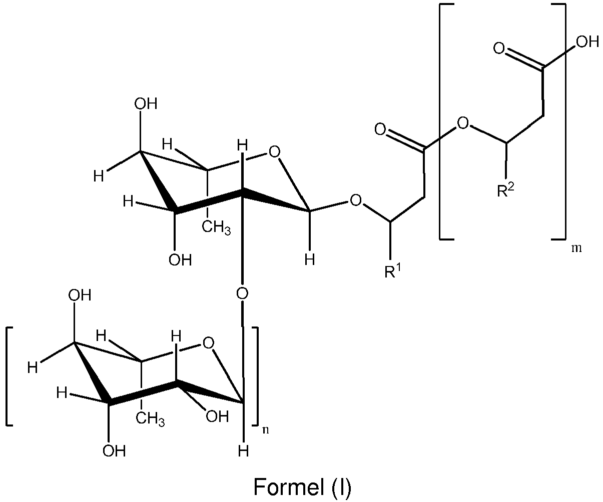 wobei
m = 2, 1 oder 0,
n = 1 oder 0,
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undecenyl und Tridecenyl und (CH₂)ₒ-CH₃ mit o = 1 bis 23, bevorzugt 4 bis 12.
Falls n = 1 ist die glykosidische Bindung zwischen den zwei Rhamnoseeinheiten bevorzugt in der α-Konfiguration. Die optisch aktiven Kohlenstoffatome der Fettsäuren liegen bevorzugt als R-Enantiomere vor (z.B. (R)-3-{(R)-3-[2-O-(α-L-rhamnopyranosyl)-α-L-rhamnopyranosyl]oxydecanoyl}oxydecanoate).

Unter dem Begriff "di-Rhamnolipid" im Zusammenhang mit der vorliegenden Erfindung werden Verbindungen der allgemeinen Formel (I) oder deren Salze verstanden, bei denen n = 1.
Unter dem Begriff "mono-Rhamnolipid" im Zusammenhang mit der vorliegenden Erfindung werden Verbindungen der allgemeinen Formel (I) oder deren Salze verstanden, bei denen n = 0.
Distinkte Rhamnolipide werden gemäß folgender Nomenklatur abgekürzt: Unter "diRL-CXCY" werden di-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.
Unter "monoRL-CXCY" werden mono-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.
Die verwendete Nomenklatur unterscheidet somit nicht zwischen "CXCY" und "CYCX". Für Rhamnolipide mit m=0 wird entsprechend monoRL-CX bzw. diRL-CX verwendet. Ist einer der oben genannten Indices X und/oder Y mit ":Z" versehen, so bedeutet dies, dass der jeweilige Rest R¹ und/oder R² = ein unverzweigter, unsubstituierter Kohlenwasserstoffrest mit X-3 bzw. Y-3 Kohlenstoffatomen aufweisend Z Doppelbindungen darstellt.
Der "pH-Wert" im Zusammenhang mit der vorliegenden Erfindung ist definiert als der Wert, welcher für die entsprechende Zusammensetzung bei 25 °C nach fünf Minuten Rühren mit einer gemäß ISO 4319 (1977) kalibrierten pH-Elektrode gemessen wird.

Alle angegebenen Prozent (%) sind, wenn nicht anders angegeben, Massenprozent. Zur Bestimmung des Gehaltes an Rhamnolipiden im Zusammenhang mit der vorliegenden Erfindung wird lediglich die Masse des Rhamnolipid-Anions, somit "allgemeinen Formel (I) abzüglich ein Wasserstoff" berücksichtigt.
Zur Bestimmung des Gehaltes an Rhamnolipiden im Zusammenhang mit der vorliegenden Erfindung werden alle Rhamnolipide durch Ansäuern in die protonierte Form (vgl. allgemeine Formel (I)) überführt und mittels HPLC quantifiziert.

Die in den erfindungsgemäßen Formulierungen enthaltenen Rhamnolipide liegen aufgrund des gegebenen pH-Wertes mindestens teilweise als Salz vor.
In erfindungsgemäß bevorzugten Zusammensetzungen sind die Kationen der enthaltenen Rhamnolipid-Salze, ausgewählt aus der Gruppe umfassend, bevorzugt bestehend aus, Li⁺, Na⁺, K⁺, Mg²⁺, Ca²⁺, Al³⁺, NH₄⁺, primäre Ammoniumionen, sekundäre Ammoniumionen, tertiäre Ammoniumionen und quaternäre Ammoniumionen.
Beispielhafte Vertreter von geeigneten Ammoniumionen sind Tetramethylammonium, Tetraethylammonium , Tetrapropylammonium, Tetrabutylammonium und [(2-Hydroxyethyl)trimethylammonium] (Cholin) sowie die Kationen von 2-Aminoethanol (Ethanolamin, MEA), Diethanolamin (DEA), 2,2',2"-Nitrilotriethanol (Triethanolamin, TEA), 1-Aminopropan-2-ol (Monoisopropanolamin), Ethylendiamin, Diethylentriamin, Triethylentetramin, Tetraethylenepentamine, 1,4-Diethylendiamin (Piperazin), Aminoethylpiperazin und Aminoethylethanolamin.
Es können auch Mischungen der vorgenannten Kationen als Kationen der enthaltenen Rhamnolipid-Salze erfindungsgemäß vorliegen.
Besonders bevorzugte Kationen, sind ausgewählt aus der Gruppe umfassend, bevorzugt bestehend aus Na⁺, K⁺, NH₄⁺ und das Triethanolammonium-Kation.
Die Gesamtmenge der vorgenannten Kationen macht bevorzugt 70 Gew.-% bis 99 Gew.-%, besonders bevorzugt 80 Gew.-% bis 90 Gew.-%, aller in der Zusammensetzung enthaltenen Kationen ohne H⁺ und H₃O⁺ aus.

Bevorzugte erfindungsgemäße Zusammensetzungen enthalten 50 Gew.-% bis 99 Gew.-%, bevorzugt 70 Gew.-% bis 95 Gew.-%, besonders bevorzugt 85 Gew.-% bis 90 Gew.-%, Rhamnolipid-Anionen, wobei sich die Gew.-% auf alle in der Zusammensetzung enthaltenen Anionen ohne OH⁻ beziehen.

In besonders bevorzugten erfindungsgemäßen Zusammensetzungen enthält die Gesamtrockenmasse 40 Gew.-% bis 98 Gew.-%, bevorzugt 50 Gew.-% bis 95 Gew.-%, besonders bevorzugt 60 Gew.-% bis 90Gew.-%, Rhamnolipide, wobei sich die Gewichtsprozente auf die Gesamttrockenmasse beziehen.

In erfindungsgemäß bevorzugten Zusammensetzungen liegen mindestens 60 Gew.%, bevorzugt mindestens 80 Gew.-%, besonders bevorzugt mindestens 95 Gew.-% der Rhamnolipide in gelöster Form vor, wobei sich die Gewichtsprozent auf die Gesamtmenge an Rhamnolipiden beziehen.
Gemessen wird dies durch HPLC Analyse des gesamten Rhamnolipids vor und nach Filtration durch einen 0,2 µm Spritzenfilter, wobei die Menge an Rhamnolipiden im Eluat der Menge gelöster Rhamnolipide entspricht.

Es ist erfindungsgemäß bevorzugt, dass die Zusammensetzungen 51 Gew.-% bis 95 Gew.-%, bevorzugt 70 Gew.-% bis 90 Gew.-%, besonders bevorzugt 75 Gew.-% bis 85 Gew.-%, diRL-C10C10 enthalten, wobei sich die Gewichtsprozente auf die Summe aller enthaltenen Rhamnolipide beziehen.
Es ist erfindungsgemäß bevorzugt, dass die Zusammensetzungen 0,5 Gew.-% bis 9 Gew.-%, bevorzugt 0,5 Gew.-% bis 3 Gew.-%, besonders bevorzugt 0,5 Gew.-% bis 2 Gew.-%, monoRL-C10C10 enthalten, wobei sich die Gewichtsprozente auf die Summe aller enthaltenen Rhamnolipide beziehen.

Bevorzugte erfindungsgemäße Zusammensetzungen sind dadurch gekennzeichnet, dass das Gewichtsverhältnis von allen enthaltenen di-Rhamnolipiden zu allen enthaltenen mono-Rhamnolipiden größer 51:49, insbesondere größer 91:9, bevorzugt größer 97:3, besonders bevorzugt größer 98:2, ist.

Es ist erfindungsgemäß bevorzugt, dass die Zusammensetzungen 0,5 bis 25 Gew.-%, bevorzugt 5 Gew.-% bis 15 Gew.-%, besonders bevorzugt 7 Gew.-% bis 12 Gew.-%, diRL-C10C12 enthalten, wobei sich die Gewichtsprozente auf die Summe aller enthaltenen Rhamnolipide beziehen.
Es ist erfindungsgemäß bevorzugt, dass die Zusammensetzungen 0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und/oder, bevorzugt und, 0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1, enthalten, wobei sich die Gewichtsprozente auf die Summe aller enthaltenen Rhamnolipide beziehen.

Besonders bevorzugte erfindungsgemäße Zusammensetzungen sind dadurch gekennzeichnet, dass sie
0,5 Gew.-% bis 15 Gew.-%, bevorzugt 3 Gew.-% bis 12 Gew.-%, besonders bevorzugt 5 Gew.-% bis 10 Gew.-%, diRL-C10C12:1,
0,5 bis 25 Gew.-%, bevorzugt 5 Gew.-% bis 15 Gew.-%, besonders bevorzugt 7 Gew.-% bis 12 Gew.-%, diRL-C10C12,
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C1 OC12:1,
enthalten, wobei sich die Gewichtsprozente auf die Summe aller enthaltenen Rhamnolipide beziehen.

Es ist überdies hinaus bevorzugt, wenn die erfindungsgemäße Zusammensetzung Rhamnolipide der Formel monoRL-CX bzw. diRL-CX in nur kleinen Mengen enthält. Insbesondere enthält die erfindungsgemäße Zusammensetzung bevorzugt
0 Gew.-% bis 5 Gew.-%, bevorzugt 0 Gew.-% bis 3 Gew.-%, besonders bevorzugt 0 Gew.-% bis 1 Gew.-%, diRLC10, wobei sich die Gewichtsprozente auf die Summe aller enthaltenen Rhamnolipide beziehen, und unter dem Begriff "0 Gew.-%" keine nachweisbare Menge zu verstehen ist.
Es ist erfindungsgemäß bevorzugt, dass die erfindungsgemäßen Zusammensetzungen im Wesentlichen frei von fettem Öl (bei 20 °C flüssigen Acylglycerolen) sind und somit insbesondere weniger als 0,5 Gew.-%, insbesondere weniger als 0,1 Gew.-%, besonders bevorzugt keine nachweisbaren Mengen, fettes Öl bezogen auf die gesamte Zusammensetzung enthalten.

Ein weiterer Gegenstand der vorliegenden Erfindung ist ein Verfahren zur Herstellung einer Lösung von Rhamnolipiden umfassend die Schritte
a) Bereitstellen einer Zusammensetzung enthaltend
   30 Gew.-% bis 70 Gew.-%, bevorzugt 35 Gew.-% bis 60 Gew.-%, besonders bevorzugt 40 Gew.-% bis 50 Gew.-%, mindestens eines Rhamnolipides wobei sich die Gewichtsprozente auf die Gesamtzusammensetzung beziehen, mit einem pH-Wert bei 25 °C von pH 1 bis pH 5, bevorzugt von pH 2,5 bis 4,0, besonders bevorzugt von pH 3,0 bis pH 3,5
b) Einstellen des pH-Wertes der Zusammensetzung auf von pH 5,5 bis pH < 7,0, bevorzugt auf von pH 5,6 bis pH 6,2, besonders bevorzugt auf von pH 5,6 bis pH 6,0 und
c) Einstellen des Wassergehaltes der Gesamtzusammensetzung auf 30 Gew.-% bis 70 Gew.-%, bevorzugt 40 Gew.-% bis 65 Gew.-%, besonders bevorzugt 50 Gew.-% bis 60 Gew.-%, Wasser
und des Rhamnolipidgehaltes der Gesamtzusammensetzung auf 30 Gew.-% bis 70 Gew.-%, bevorzugt 35 Gew.-% bis 60 Gew.-%, besonders bevorzugt 40 Gew.-% bis 50 Gew.-%, Rhamnolipide,
wobei sich die Gewichtsprozente auf die Gesamtzusammensetzung beziehen.

Erfindungsgemäß bevorzugt liegt das Rhamnolipid in der in Verfahrensschritt a) bereitgestellten Zusammensetzung mindestens teilweise dispers vor.
Unter dem Begriff "dispers vorliegen" im Zusammenhang mit der vorliegenden Erfindung, wird verstanden, dass visuell oder lichtmikroskopisch identifizierbare Rhamnolipidaggregate vorliegen, die bei Verdünnung mit Wasser unter Beibehaltung des pH-Wertes auf 10 Gew.-% Rhamnolipid im Schwerefeld der Erde sedimentieren.

Insbesondere werden in Verfahrensschritt a) Zusammensetzungen bereitgestellt, die eine Viskosität von 3 bis 15, bevorzugt von 5 bis 10, besonders bevorzugt von 6 bis 8 Pas, gemessen in einem Rheometer bei einer Scherrate von 10 s⁻¹, aufweisen.

Ein erfindungsgemäß bevorzugtes Verfahren ist dadurch gekennzeichnet, dass in Verfahrensschritt b) der pH-Wert durch Zugabe einer organischen oder anorganischen Base, bevorzugt in konzentrierter Form, eingestellt wird.

Unter dem Begriff "Base in konzentrierter Form" im Zusammenhang mit der vorliegenden Erfindung, wird verstanden, dass die Base in Form einer Zusammensetzung zugegeben wird, die mindestens 60 Gew.-%, insbesondere mindestens 80 Gew.-% Base enthält, wobei sich die Gewichtsprozente auf die zugegebene Gesamtzusammensetzung beziehen.

In dem erfindungsgemäßen Verfahren werden bevorzugt Basen ausgewählt aus der Gruppe umfassend, bevorzugt bestehend aus Alkali- und Erdalkalimetallhydroxiden wie NaOH, KOH, Mg(OH)₂, Ca(OH)₂; Al(OH)₃, NH₄OH, primäre Amine, sekundäre Amine, tertiäre Amine und quarternäre Amine eingesetzt.
Beispielhafte Vertreter von geeigneten Aminen sind 2-Aminoethanol (auch Ethanolamin, MEA), Diethanolamin (auch DEA), 2,2',2"-Nitrilotrietanol (auch Triethanolamin, TEA), 1-Aminopropan-2-ol (auch Monoisopropanolamin), [(2-Hydroxyethyl)trimethylammonium] (auch Cholin) Ethylendiamin, Diethylentriamin, Triethylentetramin, Tetraethylenepentamine, 1,4-Diethylendiamin (auch Piperazin), Aminoethylpiperazin, Aminoethylethanolamin, , Tetramethylammoniumhydroxid, Tetraethylammoniumhydroxid , Tetrapropylammoniumhydroxid, Tetrabutylammoniumhydroxid, wobei 2-Aminoethanol (auch Ethanolamin, MEA), Diethanolamin (auch DEA), 2,2',2"-Nitrilotrietanol (auch Triethanolamin, TEA), 1-Aminopropan-2-ol (auch Monoisopropanolamin) und (2-Hydroxyethyl)trimethylammonium (auch Cholin) bevorzugt eingesetzt werden. Besonders bevorzugte Basen sind NaOH, KOH, NH₃, NH₄OH und Triethanolamin. Es können auch Mischungen der vorgenannten Basen erfindungsgemäß eingesetzt werden.

Werden in dem erfindungsgemäßen Verfahren in Verfahrensschritt a) hochviskose Pasten enthaltend Rhamnolipide bereitgestellt, so können vorteilhaft in Verfahrensschritt b) und/oder c) Mischapparaturen wie z.B. Extruder eingesetzt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Charakterisierung der Produkte

### Quantifizierung von Rhamnolipiden mittels Hochleistungsflüssigchromatographie (HPLC)

Die Quantifizierung erfolgte mittels HPLC. Als Kalibrierstandard wurde jeweils die Säureform der verschiedenen Rhamnolipidspezies verwendet, die chromatographische Auftrennung erfolgte ebenfalls unter sauren Bedingungen. Dies führt dazu, dass alle RL Komponenten in eine Probe als Säureform quantifiziert werden.

Für die Probenvorbereitung wurde mit einer Verdrängerpipette (Combitip) in einem 2 ml-Reaktionsgefäß 1 ml Aceton vorgelegt und das Reaktionsgefäß zur Minimierung von Verdunstung unmittelbar verschlossen. Es folgte die Zugabe von 1 ml Rhamnolipid-haltiger Probe, die zuvor nach Bedarf verdünnt worden war. Nach Vortexen wurde die Mischung für 3 min bei 13.000 rpm abzentrifugiert, und 800 µl des Überstands in ein HPLC-Gefäß überführt.

Zum Nachweis und zur Quantifizierung von Rhamnolipiden wurde ein Evaporative Light Scattering Detektor (Sedex LT-ELSD Model 85LT) benutzt. Die eigentliche Messung wurde mittels Agilent Technologies 1200 Series (Santa Clara, Kalifornien) und der Zorbax SB-C8 Rapid Resolution Säule (4,6 x 150 mm, 3,5 µm, Agilent) durchgeführt. Das Injektionsvolumen betrug 5 µl und die Laufzeit der Methode 20 min. Als mobile Phase wurde wässrige 0,1 % TFA (Trifluor-Essigsäure, Lösung A) und Methanol (Lösung B) verwendet. Die Säulentemperatur betrug 40 °C. Als Detektoren dienen der ELSD (Detektortemperatur 60 °C) und der DAD (Diodenarray, 210 nm). Der in der Methode verwendete Gradient war:

| **t [min]** | **Lösung B vol.-%** | **Fluss [ml/min]** |
|---|---|---|
| 0,00 | 70% | 1,00 |
| 15,00 | 100% | 1,00 |
| 15,01 | 70% | 1,00 |
| 20,00 | 70% | 1,00 |

### Viskositätsmessungen

Die Messung der Viskosität erfolgt mit Hilfe eines Rheometers (MCR 302, Anton Paar Germany) in einem Platte-Platte Messsystem. Die obere Platte hatte einen Durchmesser von 40 mm, der Spaltabstand betrug 0,5 mm, Messtemperatur war 25°C. Es wurde im Scherratenbereich von 0.1 -100 s-1 gemessen.

### Beispiel 1: Herstellung einer hochkonzentrierten RL-Lösung:

Eine Fermentation mit einem die Rhamnolipid-Biosynthesegene RhIA, RHIB und RhIC enthaltenden *Pseudomonas putida* Stamm *pBBR1MCS2-Plac-rhIABC-T-Ptac-rhIC-T,* deren Herstellung in US2014296168 beschrieben ist, wurde durchgeführt. Die Vorkultur im Schüttelkolben wurde wie in WO2012013554A1 beschrieben durchgeführt. Für die Hauptkultur kam ebenfalls ein Mineralmedium (M9) zum Einsatz. Die Fermentation wurde kohlenstofflimitiert über eine Glukosezufütterung in einem 2 Liter Fermenter geführt. Die Glukosezufütterung erfolgt anhand des Gelöstsauerstoffsignals. Der Gelöstsauerstoff wurde bei 20 % Sättigung über die Rührerdrehzahl reguliert. Der pH-Wert wird über eine pH Elektrode und Zugabe von NH₄SO₄ auf 7 reguliert. Um das Überschäumen der Fermentationsbrühe zu verhindern, wurde der Entschäumer DOW Corning 1500 bei Bedarf zudosiert. Die Fermentation wurde über 4 Tage bis zu einer Biotrockenmasse von 15 g/l geführt. Die Rhamnolipidkonzentration wurde über HPLC ermittelt und betrug 9,8 g/l. Nach Abtrennen der Zellen mittels Zentrifugation bei 10.000 g wurde die Fermentationsbrühe durch Zugabe konzentrierter H₂SO₄ auf einen pH-Wert von 3,1 eingestellt. Durch erneute Zentrifugation bei 500 g wurde ein pastöses Feststoff-Konzentrat mit einem RL-Anteil von 45 Gew. % und mit einer Viskosität von > 10.000 mPas erhalten.

### Beispiel 2: Teilneutralisation mit KOH:

Unter ständigem Rühren wurde eine 50 gew.-%ige wässrige KOH Lösung zur pastösen Suspension des aufkonzentrierten Rhamnolipidpräzipitats (vgl. vorheriges Beispiel) gegeben und ein pH-Wert von 5, eingestellt. Hierbei kam es zur Verflüssigung der pastösen Masse die mit einem starken Viskositätsabfall einherging. Aus der Suspension wurde eine klare Lösung. Von dieser Lösung wurde 3 Teile abgenommen und durch weitere KOH Zugabe jeweils auf pH = 6, pH = 7 und pH = 8,5 eingestellt.

Nach Einstellung der pH-Werte der Lösungen wurde diese durch Wasserzugabe auf 50 Gew %, 40 Gew %, 30 Gew.%, 20 Gew% und 10 Gew% Rhamnolipid eingestellt und für Viskositätsmessungen verwendet. Die Ergebnisse sind in Tabelle 1 dargestellt. Es wurde festgestellt, dass durch die Teilneutralisation ein wesentlich dünnflüssigeres, aber hochkonzentriertes Produkt erhalten werde konnte. Gleichzeitig kam es bei den pH-Werten 5,6 und 6 zu einem wesentlich geringeren Viskositätsabfall beim Verdünnen der Lösungen als bei den pH-Werten 7 und 8,5.

**Tabelle 1: Viskosität (Pas, Scherrate 10 1/s) von Rhamnolipidzusammensetzungen in Abhängigkeit von Rhamnolipidkonzentration (Gew. %) und pH-Wert. Die durch * gekennzeichneten Formulierungen sind erfindungsgemäß, da hierbei die Viskosität bei hoher Konzentration gering ist und der Viskositätsabfall bei Verdünnung mit Wasser ebenfalls am geringsten ist (vergleiche die jeweiligen Viskositäten bei 10 % und 20 %).**

| | 10% | 20% | 30% | 40% | 50% |
|---|---|---|---|---|---|
| pH 3,5 | - | - | - | 6,04 | |
| pH 5,6 | 0,114 | 0,406 | 0,593* | 0,816* | 1,756* |
| pH 6,0 | 0,064 | 0,12 | 0,536* | 0,922* | 1,698* |
| pH 7,0 | 0,001 | 0,014 | 0,212 | 1,010 | 1,793 |
| pH 8,5 | 0,002 | 0,009 | 0,135 | 0,827 | 1,728 |

### Beispiel 3: Teilneutralisation mit NaOH:

Die Teilneutralisation erfolgte analog zu Beispiel 2, mit der Ausnahme, dass Anstelle von KOH eine 50 Gew. %ige NaOH Lösung verwendet wurde.

### Beispiel 4: Teilneutralisation mit NH₄OH:

Die in Beispiel 1 erhaltene, hochkonzentrierte, saure Rhamnolipidsuspension wurde durch Zugabe von Wasser auf einen Rhamnolipidgehalt von 34,6 Gew.% eingestellt, die einen pH-Wert von 3,18 hatte. Es wurden 113 g dieser Suspension entnommen und unter ständigem Rühren wurden 3,64 g einer 25 Gew. %igen NH₄OH Lösungen zugegeben. Hierbei kam es zu einer vollständigen Verflüssigung und es stellte sich ein pH-Wert von 5,63 ein.

### Beispiel 5: Teilneutralisation mit Ca(OH)₂

Zu 101 g einer sauren Rhamnolipidsuspension mit 34,6 Gew.% Rhamnolipid und einem pH-Wert von 3,18 wurden 2 g Ca(OH)₂ gegeben und die resultierende Feststoffsuspension 2 Tage bei Raumtemperatur gerührt. Hierbei kam es zu einer vollständigen Verflüssigung und es stellte sich ein pH-Wert von 5,72 ein.

### Beispiel 6: Teilneutralisation mit Mg(OH)₂

Zu 95 g einer sauren Rhamnolipidsuspension mit 34,6 Gew.% Rhamnolipid und einem pH-Wert von 3,18 wurden 1,69 g Mg(OH)₂ gegeben und die resultierende Feststoffsuspension über Nacht bei Raumtemperatur gerührt. Hierbei kam es zu einer vollständigen Verflüssigung und es stellte sich ein pH-Wert von 6,06 ein.

### Beispiel 7: Teilneutralisation mit 2-Aminoethanol (MEA)

Die Fermentation und Aufarbeitung der Rhamnolipide wurde analog zu Beispiel 1 durchgeführt, mit der Ausnahme, dass für den Fällungsschritt lediglich auf pH = 3,8 angesäuert wurde. Die erhaltene Rhamnolipidsuspension wurde durch Wasserzugabe auf einen Rhamnolipidgehalt von 40 Gew.% eingestellt und in mehrer Teile von 50 g aufgeteilt. Dann wurden unter ständigem Rühren 2,28 g Monoethanolamin (Reinheit > 99 %) zu einem der Teile zugegeben, was zu einer Verflüssigung und zum Aufklaren der Lösung führte. Der pH-Wert der erhaltenen Lösung war 6,0.

### Beispiel 8: Teilneutralisation mit Triethanolamin (TEA)

Zu 50 g einer 40 Gew.% Rhamnolipidsuspension, pH = 3,8 (vgl. Beispiel 7) wurden unter ständigem Rühren 6,8 g Triethanolamin (Reinheit > 99 %) gegeben. Dies führte zur Verflüssigung zum Aufklaren der Lösung. Der pH-Wert der erhaltenen Lösung betrug 6,2.

### Beispiel 9: Teilneutralisation mit Monoisopropanolamin (MIPA)

Zu 50 g einer 40 Gew.% Rhamnolipidsuspension, pH = 3,8 (vgl. Beispiel 7) wurden unter ständigem Rühren 2,76 g MIPA (Reinheit > 99 %)gegeben. Dies führte zur Verflüssigung zum Aufklaren der Lösung. Der pH-Wert der erhaltenen Lösung betrug 6,2.

### Beispiel 10: Teilneutralisation mit Cholinhydroxid

Zu 50 g einer 40 Gew.% Rhamnolipidsuspension, pH = 3,8 (vgl. Beispiel 7) wurden unter ständigem Rühren 9,4 g Cholinhydroxid (46 Gew.% Lösung) gegeben. Dies führte zur Verflüssigung zum Aufklaren der Lösung. Der pH-Wert der erhaltenen Lösung betrug 6,2.

## Patentansprüche

1. Zusammensetzung enthaltend
30 Gew.-% bis 70 Gew.-%, bevorzugt 35 Gew.-% bis 60 Gew.-%, besonders bevorzugt 40 Gew.-% bis 50 Gew.-%, mindestens eines Rhamnolipides und
30 Gew.-% bis 70 Gew.-%, bevorzugt 40 Gew.-% bis 65 Gew.-%, besonders bevorzugt 50 Gew.-% bis 60 Gew.-%, Wasser
wobei sich die Gewichtsprozente auf die Gesamtzusammensetzung beziehen, **dadurch gekennzeichnet, dass** der pH-Wert der Zusammensetzung bei 25 °C von 5,5 bis < 7,0 bevorzugt von 5,6 bis 6,2, besonders bevorzugt von 5,6 bis 6,0, beträgt,
wobei unter dem Begriff "Rhamnolipid" Rhamnolipide, deren protonierten Formen sowie deren Salze mitumfasst werden.

2. Zusammensetzung gemäß Anspruch 1, **dadurch gekennzeichnet, dass** die Gesamtrockenmasse 40 Gew.-% bis 98 Gew.-%, bevorzugt 50 Gew.-% bis 95 Gew.-%, besonders bevorzugt 60 Gew.-% bis 90 Gew.-%, Rhamnolipide enthält, wobei sich die Gewichtsprozente auf die Gesamttrockenmasse beziehen.

3. Zusammensetzung gemäß Anspruch 1 oder 2, **dadurch gekennzeichnet, dass** die Kationen der enthaltenen Rhamnolipid-Salze ausgewählt sind aus der Gruppe umfassend, bevorzugt bestehend aus, Li⁺, Na⁺, K⁺, Mg²⁺, Ca²⁺, Al³⁺, NH₄⁺, primäre Ammoniumionen, sekundäre Ammoniumionen, tertiäre Ammoniumionen und quaternäre Ammoniumionen.

4. Zusammensetzung gemäß mindestens einem der vorherigen Ansprüche,
**dadurch gekennzeichnet, dass** sie
50 Gew.-% bis 99 Gew.-%, bevorzugt 70 Gew.-% bis 95 Gew.-%, besonders bevorzugt 85 Gew.-% bis 90 Gew.-%, Rhamnolipid-Anionen enthält, wobei sich die Gew.-% auf alle in der Zusammensetzung enthaltenen Anionen ohne OH⁻ bezieht.

5. Zusammensetzung gemäß mindestens einem der vorherigen Ansprüche,
**dadurch gekennzeichnet, dass** sie
51 Gew.-% bis 95 Gew.-% diRL-C10C10
enthält,
wobei sich die Gewichtsprozente auf die Summe aller enthaltenen Rhamnolipide beziehen.

6. Zusammensetzung gemäß mindestens einem der vorherigen Ansprüche,
**dadurch gekennzeichnet, dass** sie
0,5 Gew.-% bis 9 Gew.-% monoRL-C10C10
enthält,
wobei sich die Gewichtsprozente auf die Summe aller enthaltenen Rhamnolipide beziehen.

7. Zusammensetzung gemäß mindestens einem der vorherigen Ansprüche,
**dadurch gekennzeichnet, dass** das Gewichtsverhältnis von allen enthaltenen di-Rhamnolipiden zu allen enthaltenen mono-Rhamnolipiden größer 51:49, insbesondere größer 91:9, bevorzugt größer 97:3, besonders bevorzugt größer 98:2, ist.

8. Zusammensetzung gemäß mindestens einem der vorherigen Ansprüche,
**dadurch gekennzeichnet, dass** sie
0,5 bis 25 Gew.-% diRL-C10C12
enthält,
wobei sich die Gewichtsprozente auf die Summe aller enthaltenen Rhamnolipide beziehen.

9. Zusammensetzung gemäß mindestens einem der vorherigen Ansprüche,
**dadurch gekennzeichnet, dass** sie
0,1 Gew.-% bis 5 Gew.-% monoRL-C10C12 und/oder, bevorzugt und
0,1 Gew.-% bis 5 Gew.-% monoRL-C10C12:1,
enthält,
wobei sich die Gewichtsprozente auf die Summe aller enthaltenen Rhamnolipide beziehen.

10. Zusammensetzung gemäß mindestens einem der vorherigen Ansprüche,
**dadurch gekennzeichnet, dass** sie
0,5 Gew.-% bis 15 Gew.-% diRL-C10C12:1,
0,5 bis 25 Gew.-% diRL-C10C12,
0,1 Gew.-% bis 5 Gew.-% monoRL-C10C12 und
0,1 Gew.-% bis 5 Gew.-% monoRL-C10C12:1,
enthält,
wobei sich die Gewichtsprozente auf die Summe aller enthaltenen Rhamnolipide beziehen.

11. Zusammensetzung gemäß mindestens einem der vorherigen Ansprüche,
**dadurch gekennzeichnet, dass** sie
0 Gew.-% bis 5 Gew.-% diRLC10
enthält,
wobei sich die Gewichtsprozente auf die Summe aller enthaltenen Rhamnolipide beziehen.

12. Verfahren zur Herstellung einer Lösung von Rhamnolipiden umfassend die Schritte
a) Bereitstellen einer Zusammensetzung enthaltend
30 Gew.-% bis 70 Gew.-%, bevorzugt 35 Gew.-% bis 60 Gew.-%, besonders bevorzugt 40 Gew.-% bis 50 Gew.-%, mindestens eines Rhamnolipides wobei sich die Gewichtsprozente auf die Gesamtzusammensetzung beziehen, mit einem pH-Wert bei 25 °C von 1,0 bis 5,0, bevorzugt von 2,5 bis 4,0, besonders bevorzugt von 3,0 bis 3,5,
b) Einstellen des pH-Wertes der Zusammensetzung auf von 5,5 bis < 7,0, bevorzugt auf von 5,6 bis 6,2, besonders bevorzugt auf von 5,6 bis 6,0, und
c) Einstellen des Wassergehaltes der Gesamtzusammensetzung auf 30 Gew.-% bis 70 Gew.-%, bevorzugt 40 Gew.-% bis 65 Gew.-%, besonders bevorzugt 50 Gew.-% bis 60 Gew.-%, Wasser und des Rhamnolipidgehaltes der Gesamtzusammensetzung auf 30 Gew.-% bis 70 Gew.-%, bevorzugt 35 Gew.-% bis 60 Gew.-%, besonders bevorzugt 40 Gew.-% bis 50 Gew.-%, Rhamnolipide,
wobei unter dem Begriff "Rhamnolipid" Rhamnolipide, deren protonierten Formen sowie deren Salze mitumfasst werden.

13. Verfahren gemäß Anspruch 12, **dadurch gekennzeichnet, dass** das Rhamnolipid in der in Verfahrensschritt a) bereitgestellten Zusammensetzung mindestens teilweise dispers vorliegt.

14. Verfahren gemäß Anspruch 12 oder 13, **dadurch gekennzeichnet, dass** in Verfahrensschritt b) der pH-Wert durch Zugabe einer organischen oder anorganischen Base, bevorzugt in konzentrierter Form, eingestellt wird.

## Claims

1. Composition comprising
30% by weight to 70% by weight, preferably 35% by weight to 60% by weight, particularly preferably 40% by weight to 50% by weight, of at least one rhamnolipid and
30% by weight to 70% by weight, preferably 40% by weight to 65% by weight, particularly preferably 50% by weight to 60% by weight, of water,
where the percentages by weight refer to the total composition, **characterized in that** the pH of the composition at 25 °C is from 5.5 to <7.0, preferably from 5.6 to 6.2 and particularly preferably from 5.6 to 6.0, wherein the term "rhamnolipids" covers rhamnolipids, protonated forms thereof and salts thereof.

2. Composition according to Claim 1, **characterized in that** the total dry mass comprises 40% by weight to 98% by weight, preferably 50% by weight to 95% by weight, particularly preferably 60% by weight to 90% by weight, of rhamnolipids, where the percentages by weight refer to the total dry mass.

3. Composition according to Claim 1 or 2, **characterized in that** the cations of the rhamnolipid salts present are selected from the group comprising, preferably consisting of, Li⁺, Na⁺, K⁺, Mg²⁺, Ca²⁺, Al³⁺, NH₄⁺, primary ammonium ions, secondary ammonium ions, tertiary ammonium ions and quaternary ammonium ions.

4. Composition according to at least one of the preceding claims, **characterized in that** said composition comprises
50% by weight to 99% by weight, preferably 70% by weight to 95% by weight, particularly preferably 85% by weight to 90% by weight, of rhamnolipid anions, where % by weight refers to all anions except OH⁻ present in the composition.

5. Composition according to at least one of the preceding claims, **characterized in that** said composition comprises
51% by weight to 95% by weight of diRL-C10C10, where the percentages by weight refer to the sum total of all rhamnolipids present.

6. Composition according to at least one of the preceding claims, **characterized in that** said composition comprises
0.5% by weight to 9% by weight of monoRL-C10C10, where the percentages by weight refer to the sum total of all rhamnolipids present.

7. Composition according to at least one of the preceding claims, **characterized in that** the weight ratio of all di-rhamnolipids present to all mono-rhamnolipids present is greater than 51:49, particularly greater than 91:9, preferably greater than 97:3, particularly preferably greater than 98:2.

8. Composition according to at least one of the preceding claims, **characterized in that** said composition comprises
0.5 to 25% by weight of diRL-C10C12,
where the percentages by weight refer to the sum total of all rhamnolipids present.

9. Composition according to at least one of the preceding claims, **characterized in that** said composition comprises
0.1% by weight to 5% by weight of monoRL-C10C12 and/or, preferably and
0.1% by weight to 5% by weight of monoRL-C10C12:1, where the percentages by weight refer to the sum total of all rhamnolipids present.

10. Composition according to at least one of the preceding claims, **characterized in that** said composition comprises
0.5% by weight to 15% by weight of diRL-C10C12:1,
0.5 to 25% by weight of diRL-C10C12,
0.1% by weight to 5% by weight of monoRL-C10C12 and 0.1% by weight to 5% by weight of monoRL-C10C12:1,
where the percentages by weight refer to the sum total of all rhamnolipids present.

11. Composition according to at least one of the preceding claims, **characterized in that** said composition comprises
0% by weight to 5% by weight of diRLC10,
where the percentages by weight refer to the sum total of all rhamnolipids present.

12. Method for preparing a solution of rhamnolipids comprising the steps of
a) providing a composition comprising
30% by weight to 70% by weight, preferably 35% by weight to 60% by weight, particularly preferably 40% by weight to 50% by weight, of at least one rhamnolipid, where the percentages by weight refer to the total composition, and having a pH at 25°C of 1.0 to 5.0, preferably of 2.5 to 4.0, particularly preferably of 3.0 to 3.5,
b) adjusting the pH of the composition to 5.5 to < 7.0, preferably to 5.6 to 6.2, particularly preferably to 5.6 to 6.0, and
c) adjusting the water content of the total composition to
30% by weight to 70% by weight, preferably 40% by weight to 65% by weight, particularly preferably 50% by weight to 60% by weight, of water
and adjusting the rhamnolipid content of the total composition to 30% by weight to 70% by weight, preferably 35% by weight to 60% by weight, particularly preferably 40% by weight to 50% by weight, of rhamnolipids, wherein the term "rhamnolipids" covers rhamnolipids, protonated forms thereof and salts thereof.

13. Method according to Claim 12, **characterized in that** the rhamnolipid in the composition provided in method step a) is present at least partially dispersed.

14. Method according to Claim 12 or 13, **characterized in that** the pH is adjusted in method step b) by adding an organic or inorganic base, preferably in concentrated form.

## Revendications

1. Composition contenant
30 % en poids à 70 % en poids, de préférence 35 % en poids à 60 % en poids, de façon particulièrement préférée 40 % en poids à 50 % en poids, d'au moins un rhamnolipide et 30 % en poids à 70 % en poids, de préférence 40 % en poids à 65 % en poids, de façon particulièrement préférée 50 % en poids à 60 % en poids, d'eau
les pour cent en poids se rapportant à la composition totale,
**caractérisée en ce que** le pH de la composition à 25 °C vaut de 5,5 à < 7,0, de préférence de 5,6 à 6,2, de façon particulièrement préférée de 5,6 à 6,0,
sous le terme « rhamnolipide » étant englobés les rhamnolipides, leurs formes protonées ainsi que leurs sels.

2. Composition selon la revendication 1, **caractérisée en ce que** la matière sèche totale contient 40 % en poids à 98 % en poids, de préférence 50 % en poids à 95 % en poids, de façon particulièrement préférée 60 % en poids à 90 % en poids de rhamnolipides, les pour cent en poids se rapportant à la matière sèche totale.

3. Composition selon la revendication 1 ou 2, **caractérisée en ce que** les cations des sels de rhamnolipides contenus sont choisis dans le groupe comprenant, de préférence consistant en, Li⁺, Na⁺, K⁺, Mg²⁺, Ca²⁺, Al³⁺, NH₄⁺, des ions ammonium primaire, des ions ammonium secondaire, des ions ammonium tertiaire et des ions ammonium quaternaire.

4. Composition selon au moins l'une quelconque des revendications précédentes, **caractérisée en ce qu'**elle contient
50 % en poids à 99 % en poids, de préférence 70 % en poids à 95 % en poids, de façon particulièrement préférée 85 % en poids à 90 % en poids, d'anions de rhamnolipides, les % en poids se rapportant à tous les anions contenus dans la composition hormis OH⁻.

5. Composition selon au moins l'une quelconque des revendications précédentes, **caractérisée en ce qu'**elle contient
51 % en poids à 95 % en poids de diRL-C10C10 les pour cent en poids se rapportant à la somme de tous les rhamnolipides contenus.

6. Composition selon au moins l'une quelconque des revendications précédentes, **caractérisée en ce qu'**elle contient
0,5 % en poids à 9 % en poids de monoRL-C10C10 les pour cent en poids se rapportant à la somme de tous les rhamnolipides contenus.

7. Composition selon au moins l'une quelconque des revendications précédentes, **caractérisée en ce que** le rapport pondéral de tous les di-rhamnolipides contenus à tous les mono-rhamnolipides contenus est supérieur à 51:49, en particulier supérieur à 91:9, de préférence supérieur à 97:3, de façon particulièrement préférée supérieur à 98:2.

8. Composition selon au moins l'une quelconque des revendications précédentes, **caractérisée en ce qu'**elle contient
0,5 à 25 % en poids de diRL-C10C12
les pour cent en poids se rapportant à la somme de tous les rhamnolipides contenus.

9. Composition selon au moins l'une quelconque des revendications précédentes, **caractérisée en ce qu'**elle contient
0,1 % en poids à 5 % en poids de monoRL-C10C12 et/ou, de préférence et
0,1 % en poids à 5 % en poids de monoRL-C10C12:1,
les pour cent en poids se rapportant à la somme de tous les rhamnolipides contenus.

10. Composition selon au moins l'une quelconque des revendications précédentes, **caractérisée en ce qu'**elle contient
0,5 % en poids à 15 % en poids de diRL-C10C12:1,
0,5 à 25 % en poids de diRL-C10C12,
0,1 % en poids à 5 % en poids de monoRL-C10C12 et
0,1 % en poids à 5 % en poids de monoRL-C10C12:1,
les pour cent en poids se rapportant à la somme de tous les rhamnolipides contenus.

11. Composition selon au moins l'une quelconque des revendications précédentes, **caractérisée en ce qu'**elle contient
0 % en poids à 5 % en poids de diRL-C10,
les pour cent en poids se rapportant à la somme de tous les rhamnolipides contenus.

12. Procédé pour la préparation d'une solution de rhamnolipides, comprenant les étapes
a) disposition d'une composition contenant 30 % en poids à 70 % en poids, de préférence 35 % en poids à 60 % en poids, de façon particulièrement préférée 40 % en poids à 50 % en poids, d'au moins un rhamnolipide, les pour cent en poids se rapportant à la composition totale, ayant un pH à 25 °C de 1,0 à 5,0, de préférence de 2,5 à 4,0, de façon particulièrement préférée de 3,0 à 3,5,
b) ajustement du pH de la composition à une valeur de 5,5 à < 7,0, de préférence à une valeur de 5,6 à 6,2, de façon particulièrement préférée à une valeur de 5,6 à 6,0, et
c) ajustement de la teneur en eau de la composition totale à une valeur de 30 % en poids à 70 % en poids, de préférence de 40 % en poids à 65 % en poids, de façon particulièrement préférée de 50 % en poids à 60 % en poids, d'eau et de la teneur en rhamnolipides de la composition totale à une valeur de 30 % en poids à 70 % en poids, de préférence de 35 % en poids à 60 % en poids, de façon particulièrement préférée de 40 % en poids à 50 % en poids, de rhamnolipides,
sous le terme « rhamnolipide » étant englobés les rhamnolipides, leurs formes protonées ainsi que leurs sels.

13. Procédé selon la revendication 12, **caractérisé en ce que** dans la composition disposée dans l'étape a) du procédé le rhamnolipide se trouve au moins en partie sous forme dispersée.

14. Procédé selon la revendication 12 ou 13, **caractérisé en ce que** dans l'étape b) du procédé on ajuste le pH par addition d'une base organique ou inorganique, de préférence sous forme concentrée.
